# EUROPEAN PATENT SPECIFICATION

(11) **EP 0 316 453 B1**
(45) Date of publication and mention of the grant of the patent: **27.03.1996**
(21) Application number: 88904645.4
(22) Date of filing: 27.05.1988
(51) Int. Cl.: G01N 33/48, G01N 33/49

(54) **METHOD FOR CLASSIFYING LEUKOCYTES AND REAGENTS**
VERFAHREN ZUM KLASSIFIZIEREN VON LEUKOZYTEN UND REAGENZIEN
PROCEDE DE CLASSIFICATION DE LEUCOCYTES ET REACTIFS UTILISES

(30) Priority: 29.05.1987 JP 134433/87
(43) Date of publication of application: 24.05.1989
(73) Proprietor: TOA MEDICAL ELECTRONICS CO., LTD., Hyogo-ku Kobe-shi Hyogo-ken (JP)
(72) Inventor: HAMAGUCHI,Yukio, Hyogo-ku Kobe-shi Hyogo 652 (JP); ITO, Kenji Toa Medical Electronics Co., Ltd., Hyogo-ku Kobe-shi Hyogo 652 (JP); TSUJINO, Yukio Toa Medical Electronics Co., Ltd., Hyogo-ku Kobe-shi Hyogo 652 (JP); MORIYAMA,Kazuhiro Toa Med. Electr. Co. Ltd, Hyogo-ku Kobe-shi Hyogo 652 (JP); TAKENAKA,Ikuya Toa Med. Electr. Co. Ltd., Hyogo-ku Kobe-shi Hyogo 652 (JP); MORIKAWA,Takashi Toa Med. Electr. Co. Ltd., Hyogo-ku Kobe-shi Hyogo 652 (JP); OHMI,Hitomi Toa Med. Electr. Co. Ltd., Hyogo-ku Kobe-shi Hyogo 652 (JP)
(74) Representative: Lehn, Werner, Dipl.-Ing.
(86) International application number: JP8800514
(87) International publication number: WO8809504

(56) References cited:
- EP-A- 0 003 340
- EP-A- 0 177 137
- WO-A-84/03771
- JP-A- 6 073 356
- JP-A- 6 179 163
- JP-A- 6 271 857
- US-A- 4 099 917

## Description

The present invention relates to a method of classifying and measuring blood corpuscles in the area of clinical testing. More particularly, the present invention relates to a method of and reagents for classifying, differentiating and counting leukocytes in blood.

Leukocytes in the peripheral blood of normal subjects consist of five types, i.e., lymphocytes, monocytes, neutrophils, eosinophils and basophils. The latter three kinds of leukocytes are collectively referred to as granulocytes. Different leukocyte types have different functions and counting of leukocytes in the blood according to their type provides valuable information for diagnostic purposes. For instance, an increase in the number of neutrophils is associated with such diseases as inflammations, myocardial infarction and leukemia, and a decrease in their number is associated with viral diseases, hypoplastic anemia, agranulocytosis, etc. On the other hand, an increase in the number of eosinophils is found in such diseases as parasitosis, Hodgkin's disease and allergosis. An increased number of monocytes occurs either during the convalescence period of patients suffering from infections diseases or in such diseases as monocytic leukemia.

In addition to the above-mentioned lymphocytes, monocytes, neutrophils, eosinophils and basophils which are generally referred to as "normal cells", abnormal cells occasionally appear in the peripheral blood of patients suffering from certain hemodyscrasias. For instance, gemmules or blasts are sometimes found in the peripheral blood of leukemia patients. Such abnormal cells also occur in various types and classifying them and determining the number of cells in each class of abnormal cells is also of great importance in clinical fields.

The classification and counting of leukocytes has most commonly been conducted by the differential counting method which is also referred to as the visual counting method, or simply as the manual technique.

In this method, a blood sample is smeared on a glass plate and the blood corpuscles in the smear are stained for examination by microscopy. The technician identifies the type of individual leukocytes according to their morphological features (e.g., their size, the morphology of their nucleus and cytoplasm, and the presence or absence of granules) or the degree of dye uptake and performs classification and counting of them. At ordinary laboratories, 100 - 200 leukocytes are usually counted for each sample and the percentage of the total leukocyte count occupied by each type of corpuscle is recorded as a measured value.

The differential counting method has several disadvantages. First, microscopic observation must be preceded by cumbersome procedures for preparing a specimen that involve such steps as smearing a blood sample on a glass plate, fixing the corpuscles and staining them. Secondly, it is a great burden for the technician to identify subtle differences between corpuscles by microscopic classification and counting. Thirdly, it is difficult even for a skilled technician to yield consistent counts by the manual method since, aside from the small number of leukocytes computed, the smeared sample often has an uneven distribution of blood corpuscles.

Under these circumstances, there has arisen a strong need for a method to be developed that is capable of automated classification and counting of leukocytes. The automated techniques so far realized may be roughly divided into two types.

The first method consists of recording the images of corpuscles with a video camera or some other suitable imaging device and classifying the leukocytes by means of image processing on a computer. The operating principles of 5 this method are similar to those of the conventional visual counting method but primarily due to the existence of many corpuscles that defy classification by processing with a computer, this method has not yet become an ideal alternative to the manual method. Another problem with this method is that it requires sophisticated equipment which is bulky and costly.

The other approach toward automatic classification and counting of leukocytes is based on a flow system. In this method, a blood sample having corpuscles suspended in a diluent is permitted to flow in such a way that the corpuscles will individually (one by one) pass through a constricted detector and leukocyte classification is conducted by analyzing the signals generated by the detector. This second method which makes use of a flow system is further subdivided into two categories.

In a method of the first category, an electrolyte in which all red cells present are disrupted with a lysing agent so that only leukocytes will be suspended is permitted to flow through an orifice and the change in electrical impedance that occurs at the orifice when each corpuscle passes through it is detected, the magnitude of the detected signal being used as a basis for classification of leukocytes.

A method of the second category is characterized by the use of a flow cytometer that comprises a light source, a flow cell that permits the blood cells in a sample to flow one by one through a constricted channel, a photometric unit that detects light issuing from each blood cell, and an analyzer for analyzing the detected signal. In this method, the corpuscles in the sample which are stained are illuminated under light and the fluorescence emitted from the irradiated corpuscles is detected, optionally together with scattered light, with leukocyte classification being conducted in accordance with the intensity of the detected signal.

This method of the second category has problems in practical use such as the need to adopt a complicated staining process, as well as the use of sophisticated and costly equipment including an optical system.

One of the principles underlying the method of the first category is disclosed in JP-B- 508,789 and US-A- 3,390,326. According to this principle, a sample prepared by suspending particles in a fluid medium having a different dielectric constant is allowed to pass through a fluid channel having a constricted portion held between closely adjacent electrodes and the change that occurs in the electric impedance between the electrodes on account of the difference in dielectric constant between the particles and the fluid medium is detected.

A description of a practical apparatus that operates on this principle may be found in Ichiro Kurokawa et al., "Toa jidokekkyukeisuki no shiyoukeiken (Experience of the Use of Toa Automatic Blood Cell Counter)" in "Rinsho Byori (Clinical Pathology)", vol. 16, pp. 251 - 255, 1968. This apparatus has a 3.5 MHz high-frequency oscillator which applies a high-frequency current to the detector circuit, and the change in electric impedance that occurs between the electrodes in the detector circuit as corpuscles in suspension pass through the detector circuit is detected.

A description of leukocyte measurements with a Toa Automatic Blood Cell Counter may be found in Ichiro Kurokawa et al., "Rinsho Kensa (Clinical Testing)", vol. 11, pp. 148 - 151, 1967. The described method consists of adding saponin to a suspension of blood cells so that the erythrocytes are lysed to enable the measurement of leukocytes that are left intact.

Besides saponin, there are several other lysing agents available that are capable of lysing erythrocytes. Among such blood lysing agents, CTAC (cetyltrimethyl ammonium chloride) and Tergitol nonionic NPX® display a strong lytic activity under use conditions but, at the same time, the protoplasm of leukocytes is also attacked and their nuclei will become almost naked. In contrast, saponin allows leukocytes to remain fairly close to their intact state. Therefore, CTAC and Tergitol nonionic NPX® are unsuitable for use as blood lysing agents in a model such as Toa Automatic Red Cell Counter that detects the change in electric impedance at high frequencies (see Kazuo Shintani, "Hakekkyusantei no mondaiten (Problems with Leukocyte Counting)" in "Rinsho Kensa (Clinical Testing)", vol. 12, pp. 900 - 905, 1968.

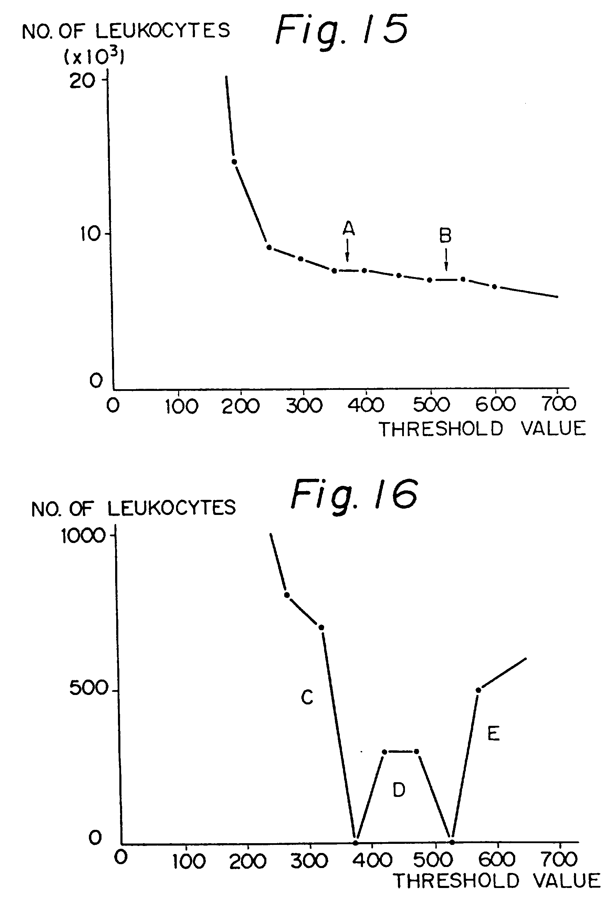

In determining the number of leukocytes with an automatic blood cell counter, it is necessary that the magnitude of signals from leukocytes be sufficiently large compared to the signals of dissolved erythrocyte membranes (ghosts) and attendant noise to enable clear differentiation between the two kinds of signals. In order to determine whether this condition is established, a cumulative size-frequency distribution curve as shown in Fig. 15 is often used. The graph shown in Fig. 15 is constructed by plotting the signal threshold values of an automatic blood cell counter on the horizontal axis, and the number of detected signals exceeding a certain signal threshold value on the vertical axis. In Fig. 15, portion A is generally referred to as "a flat portion" of the cumulative size-frequency distribution curve, and in order to ensure that leukocyte signals being measured are sufficiently larger than the above-mentioned noise and ghost signals to yield consistent leukocyte counts, the equipment and reagents used therewith must be adjusted so as to allow the flat portion A to be extended.

In practice, however, it has been pointed out that measurements of leukocytes with a Toa Automatic Blood Cell Counter using saponin as a blood lysing agent produce a shorter "flat portion" than when the DC method to be described later in this specification is employed. For instance, the graph in Fig. 15 was constructed from the data in Table 1 given in Yuji Takamori et al., "Hakekkyuryudobunpu no hendoyoin to sono keisuchi ni oyobosu eikyo ni tsuite (On Factors Causing Variations in Leukocyte Size-Frequency Distribution and Their Effects on Leukocyte Counts)" in MCC News, No. 34, pp. 12 - 21, Toa Tokushu Denki, Hyogo, Japan, 1969 (the data shows the cumulative size-frequency distribution obtained with saponin added to samples left for 5 minutes after dilution), and the flat portion of the curve in this graph extends only from the threshold value of 350 to 400.

As for the shortness of the flat portion obtained when leukocyte measurements are conducted with a Toa Automatic Red Cell Counter using saponin, P.W. Helleman et al., Scand. J. Haemat., 6, pp 160 - 165, 1969 comments that this phenomenon would be due to the difference in dielectric properties between dissimilar types of leukocytes (as between a lymphocyte and a granulocyte).

A careful review of the cumulative size-frequency distribution curve in Fig. 15 will show that it contains a second flat portion B on the right side of the first flat portion A. This fact will become clearer if one constructs a size-frequency distribution curve as shown in Fig. 16 by plotting the number of leukocytes for each threshold level as calculated from the cumulative size-frequency distribution curve in Fig. 15. Stated more specifically, Fig. 16 contains the population of erythrocyte ghosts and noise which is indicated by C, the first population of leukocytes indicated by D which lies rightward of C, and the second population of leukocytes indicted by E which is situated rightward of D. It is therefore possible to say that the division of the leukocyte size-frequency distribution into two populations has produced short flat portions in the cumulative size-frequency distribution.

The foregoing discussion amounts to a showing of the fact that two different populations of leukocytes have to date been classified and counted by the Toa Automatic Blood Cell Counter and that the above-described method of detecting the change in electric impedance at high frequency (this method is hereinafter referred as the RF method) has the potential to count leukocytes after classifying and diffferentiating them into several types.

However, at the time when the Toa Automatic Blood Cell Counter was developed and commercialized, the primary concern was to obtain leukocyte counts in a consistent and reliable manner and no detailed investigation was conducted to unravel the reason for the shortness of flat portions of a cumulative size-frequency distribution curve. On the contrary, most efforts were directed at adjusting the equipment and reagents in such a way that the flat portions could be extended as much as possible to ensure consistent counting of leukocytes.

Besides the RF method described above, the principle underlying the method of the first category which is included in the scope of the methods making use of a flow system is also described in JP-B- 217,947 and US-A- 2,656,508. According to this principle, a sample having particles suspended in a fluid medium having a different conductivity is allowed to pass through a narrow current channel and any change in current that occurs on account of the difference in conductivity between the particles and the fluid medium is detected. This method is hereunder referred to as the DC method. In the DC method, the magnitude of a signal detected is substantially proportional to the volume of particles.

When this DC method is combined with the already-described RF method, information on the volume of particles is obtained by the DC method and, in addition to that, information derived from the structure of the particles and the properties of the constituent materials of the particles can be obtained. An apparatus that relies on this approach for classifying several populations of different types of particles from a system comprising a mixture of different types of particles in the same suspension is disclosed in JP-B-785,859 and US-A-3,502,974. However, these patents show nothing about the possibility of classifying and counting different populations of leukocytes.

The already-described RF method may be modified in such a way that, instead of disrupting corpuscles, their contents are replaced so that their dielectric constant is changed, followed by classification and counting procedures. A method based on this approach is disclosed in JP-B- 936,823 and US-A- 3,836,849. In the second example of these patents, 250 µl of a 1% saponin solution is added to 100 µl of whole blood suspended in a phosphate buffered physiological saline solution having a pH of 7.2, and then the erythrocytes are lysed and two distinct peaks for leukocytes appear. This result agrees well with that obtained by performing measurements with a Toa Automatic Blood Cell Counter (Fig. 16).

Even if the RF method is performed using saponin which produces a comparatively mild action on corpuscles, the protoplasm of leukocytes is slowly disrupted to cause gradual attenuation of signals from leukocytes. If one wants to replace the contents of corpuscles at normal pHs without disrupting them as in JP-B- 936,823, it is necessary to reduce the concentration of saponin to a significantly low level so that it will act slowly on leukocytes. However, in this case, the preliminary treatment for starting a measurement takes quite a long time as compared to the time of the preliminary treatment necessary for ordinary leukocyte measurements (3 - 5 minutes), so it is not suitable for practical purposes to use this method with automatic analyzers which have to process many specimens in a short period of time.

A different approach that brings about a change in leukocytes in a fairly short period of time and which classifies the leukocytes into three populations on the basis of the amount of that change has been realized using the DC method and is described in National Publication of Translated Version No. 500097/1985 and US-A-4,485,175.

In this method, a quaternary ammonium salt which is also described in Japanese Patent No. 936,823 is used as a cytolytic agent and by using it at low concentration, a change in the volume of leukocytes is produced and the leukocytes are classified into three populations on the basis of this difference. However, the results of an example given in US-A- 4,485,175 are as shown in Fig. 17 and lymphocytes, monocytes and granulocytes in the three populations of leukocytes are not necessarily completely separated or differentiated.

Furthermore, the quaternary ammonium salt, even if it is used at low concentration, has such a great effect on corpuscles that hemolysis will take place within a short period of time. According to Japanese Patent No. 936,823, only the dielectric constants of corpuscles are changed without replacing or disrupting their contents, but in the absence of any specific data, it is not clear whether this is actually possible.

Considering the fact that quaternary ammonium salts cause an undesirably high degree of damage to leukocytes that are to be classified and counted, National Publication of Translated Version No. 502277/1986 and W085/05684 propose a method in which mildly acting saponin is added at high concentration and its lysing action is quenched at the time when the erythrocytes have been lysed and only thereafter is analysis performed with a flow cytometer or by the combination of the RF and DC methods.

This method requires a fixing agent in order to quench the lysing action and, furthermore, a special procedure such as adding it at a predetermined timing and thereafter heating the cells at elevated temperatures must be taken. This method is therefore not very effective for use with automatic analyzers.

As described above, among the methods that automatically classify and count leukocytes using a flow system, the method of the second category which makes use of a flow cytometer has a disadvantage in that the equipment is sophisticated and expensive. As for the method of the first category which detects the change in impedance that occurs at an orifice when corpuscles pass through the orifice, and wherein leukocytes are classified in accordance with the magnitude of detected signals, certain problems also arise depending on whether leukocytes are changed mildly or violently: in the former case, it takes much time to complete the preliminary treatments or a special fixing agent must be added at a predetermined timing or subsequent heat treatment is necessary; in the latter case, leukocytes are damaged so extensively that they cannot be classified into more than three types. Therefore, none of the methods of the second category that have so far been proposed are satisfactory for practical purposes. As for the method of the first category, no technique has ever existed that is capable of classifying and counting abnormal cells such as the already-described blasts by utilizing this method.

Furthermore, saponin as a cytolytic agent to be used in the method of the first category is labile in terms of blood lysing action whereas quaternary ammonium salts are too violent as noted above.

JP-A-61-79163 discloses a reagent for the determination of reticulosytes comprising a spheroidizing agent, a fixing agent which is an aldehyde and a basic fluorescence dye. As a spheroidizing agent of erythrocytes a cationic or nonionic surfactant is used.

EP-A-0 177 137 discloses a reagent for the determination of basophils which comprises a surfactant and a dilute acid. By the use of this reagent, all leukocytes with the exception of basophils are destroyed. Basophils remain intact as the only whole cells observable in the treated sample. Thus, the reagent of this reference enables the counting of basophils only, and not of any other leukocyte groups.

EP-A-0 003 340 discloses a reagent for the determination of haemoglobin, comprising a surfactant in an aqueous alkaline solution. There is no disclosure with regard to a reagent and a method for classifying leukocytes.

WO-A-84/03771 discloses a reagent for the determination of leukocytes comprising an aqueous solution of quartenary ammonium salts and at least one cationic surfactant. This surfactant is added as a supplementary ingredient for adjusting the lysing ability of the quartenary ammonium salts.

According to the present invention, neither saponin nor quaternary ammonium salts are used as cytolytic agents, and instead a cytolytic agent having consistent lysing action is used to lyse erythrocytes and damage leukocytes in a very short period of time. Thereafter, analysis is made by the combination of the already-described DC and RF methods, thereby providing remarkable advantages, such as classification of normal cells into five types as well as classification of abnormal cells, which have been unattainable in the prior art techniques of leukocyte classification.

In order to solve the aforementioned problems of the prior art and to realize certain remarkable advantages, the present invention provides reagents and methods for classifying leukocytes as set forth below.

According to a first aspect the present invention provides a
reagent for classifying leukocytes in a blood sample that lyses erythrocytes and which acts on leukocytes to enable the classification and counting of leukocytes, said reagent being composed of:
(a) first fluid which is an agent for diluting blood and which contains a hyperosmotic agent;
(b) a second fluid that contains a surfactant and which is to be added to a sample of blood that has been diluted with the first fluid, said surfactant being selected from the group consisting of:
   (i) a surfactant of a first group which is a polyoxyethylene-based anionic surfactant represented by the formula:

      R₁-R₂-(CH₂CH₂O)ₙ-X

      where R₁ is an alkyl, alkenyl, or alkynyl group having 10-22 carbon atoms; 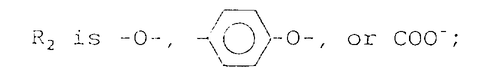 n is an integer of 8-30
      X is -SO₃Na, COONa, OSO₃Na or ONa; and
   (ii) a surfactant of a second group which is a polyoxyethylene-based nonionic surfactant represented by the formula:

      R₁-R₂-(CH₂CH₂O)ₙ-H

      where R₁ is an alkyl, alkenyl, or alkynyl group having 10-22 carbon atoms; 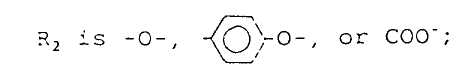 n is an integer of 8-30;
      the first or second fluid further containing a buffer agent.

Preferred embodiments thereof are described in the claims 2 and 3.

According to a second embodiment the present invention provides a reagent for classifying leukocytes in a blood sample that lyses erythrocytes and which acts on leukocytes to enable the classification and counting of leukocytes, said reagent consisting essentially of a buffer agent, a solubilizing agent that selectively reduces the size of monocytes in leukocytes and at least one surfactant as defined in claim 1. This reagent may also contain a hyperosmotic agent.

The solubilizing agent is at least one member selected from the group consisting of the following:
solubilizing agents of a first group (urea-based):

| | |
|---|---|
| urea | thiourea |
| 1,1-dimethylurea | ethylene urea |
| methylurethane | 1,3-dimethylurea |
| urethane (H₂NCOOC₂H₅) | |

solubilizing agents of a second group:
n-octyl β-D-glucoside
CHAPS (3-[(3-cholamidopropyl)dimethylammonio]-1-propanesulfonate)
CHAPSO (3-[(3-ch ol amidopropyl)dimethylammonio]-2-hydroxy-1-propanesulfonate)
MEGA 8, 9, 10 (octanoyl-, nonanoyl- or decanoyl-N-methylglucamide)
sucrose monocaprate
N-formylmethylleucylalanine

solubilizing agents of a third group (guanidines):

| | |
|---|---|
| guanidine thiocyanate | guanylguanidine |
| guanidine hydrochloride | guanidine rhodanate |
| guanidine nitrate | 1,1,3,3-tetraguanidine |
| guanidine carbonate | |
| guanidine phosphate | |
| guanidine sulfate | |

solubilizing agents of a fourth group (bile salts):
sodium deoxycholate
taurocholic acid
cholic acid

solubilizing agents of a fifth group (halogen-substituted acetic acids):
sodium trichloroacetate
sodium tribromoacetate
sodium dichloroacetate
sodium dibromoacetate
sodium monochloroacetate
sodium monoboromoacetate.

According to a third aspect the present invention provides a reagent for classifying leukocytes which is composed of the following two fluids (a) and (b):
(a) a first fluid as defined in claim 1; and
(b) a second fluid that contains the reagent as set forth in any one of claims 4 to 6 and which is to be added to a blood sample that has been diluted with the first fluid.

This reagent may also contain a surfactant in the first fluid.

Furthermore, the present invention provides a method of classifying leukocytes into lymphocytes, monocytes and granulocytes as defined in claim 9. A method of classifying leukocytes into four types, lymphocytes, monocytes, eosinophils, basophils and neutrophils is as defined in claim 12.

Abnormal cells may be detected by the RF and DC particle assay techniques by use of the reagents of the invention.

By using the reagents of the present invention for classifying leukocytes, the erythrocytes in blood are lysed and the leukocyte species are individually damaged. When the so treated sample is subjected to a measurement by the combination of the RF and DC methods, the leukocytes are separated into three populations of lymphocytes, monocytes and granulocytes in accordance with the difference in speed at which the cell volume changes depending upon the amount of damage sustained by individual leukocytes. In accordance with the present invention, two-dimensional information is obtained by the RF and DC methods and the reagents of the present invention have characteristic actions; because of these features, the present invention enables leukocytes to be classified into three types in a more efficient and reliable way than the method described in US-A-4,485,175 which uses a quaternary ammonium salt as a cytolytic agent and which classifies leukocytes into three types by the DC method alone.

If the reagents for leukocyte classification of the present invention are used and a measurement made by the combination of the RF and DC methods, the leukocytes are separated into four populations of lymphocytes, monocytes, eosinophils and granulocytes other than eosinophils in accordance with the difference in speed at which the cell volume changes in dependence upon the amount of damage sustained by individual leukocytes. In this case, the RF and DC methods may be combined in various ways, and one effective way is that the leukocytes are first classified into three types, lymphocytes, monocytes and granulocytes, by the RF and DC methods, and then the eosinophils that selectively remain intact after the passage of a predetermined period of time are counted by both the RF and DC methods or by the DC method alone.

In another embodiment, the leukocytes are classified into four types by the method described above, while at the same time, the basophils are selectively left intact using a reagent for the measurement of basophils and the sample obtained is subjected to measurement by both the RF and DC methods or by the DC method alone. As a result of these procedures, the numbers of eosinophils and basophils are calculated, so the number of neutrophils left in the granulocytes can be determined by subtracting the numbers of eosinophils and basophils from the total count of granulocytes. In this way, the leukocytes can be classified into five types, with the respective numbers of lymphocytes, monocytes, neutrophils, eosinophils and basophils in the leukocytes as well as their percentages being established.

The methods of the present invention, unlike the method described in National Publication of Translated Version No. 502277/1986, have no need to use a labile lytic agent, saponin, nor do they require treatment with a special fixing agent or heat treatment at elevated temperatures; therefore, these methods find particular advantages in use with automatic analyzers.

The characteristic actions of the reagents for leukocyte classification of the present invention are described below.

The surfactant of the first group contained in the reagents of the present invention is an anionic surfactant, and the surfactant of the second group is a nonionic surfactant. In general application, anionic and nonionic surfactants are both used extensively as detergents, emulsifiers, dispersants and penetrants in various industrial fields including textiles, laundry, synthetic resins, printing inks, metals, photography, foodstuffs and pharmaceuticals.

In contrast, the quaternary ammonium salt used as a cytolYtic agent in methods such as the one described in US-A-4,488,175 is a cationic surfactant. In the general applications of cationic surfactants, properties based on their surface activity such as wetting property, detergency and ability to lower surface tension are rarely applied directly, and they are extensively and chiefly used as bactericides, disinfectants, water-proofing agents, softening agents, antistats and sealing agents in such industrial fields as textiles, rubbers, plastics, pharmaceuticals, civil engineering, ceramics and petroleum.

Saponin has often been used as a cytolytic agent and unlike the above-described anionic, nonionic and cationic synthetic surfactants, saponin is a natural chemical substance of plant origin, the chemical structure and properties of which will vary from one production lot to another.

As such, these cytolytic agents have different chemical properties and different origins, so they naturally cause different actions on leukocytes.

Of the cytolytic agents listed above, saponin is the mildest in its action on cells whereas the quaternary ammonium salt damages cells most violently. In comparison, the strength of the action of anionic and nonionic surfactants on cells would be intermediate between saponin and the quaternary ammonium salt.

A comparison of the amounts of cell damage caused by different cytolytic agents is given in Table 1 below in terms of the size of lymphocytes. The figures in the table are approximate values.

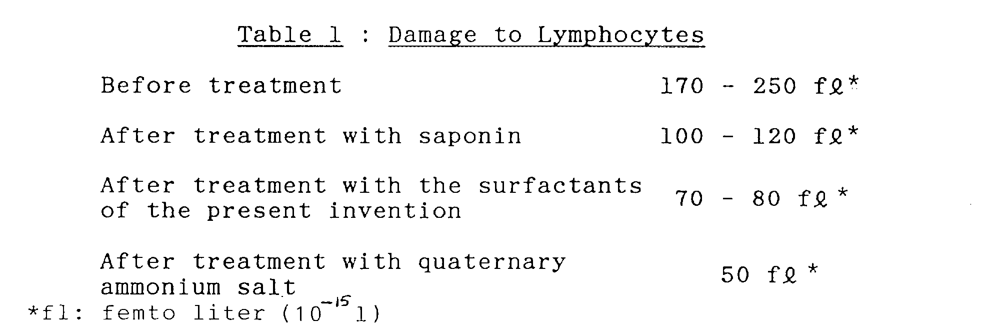

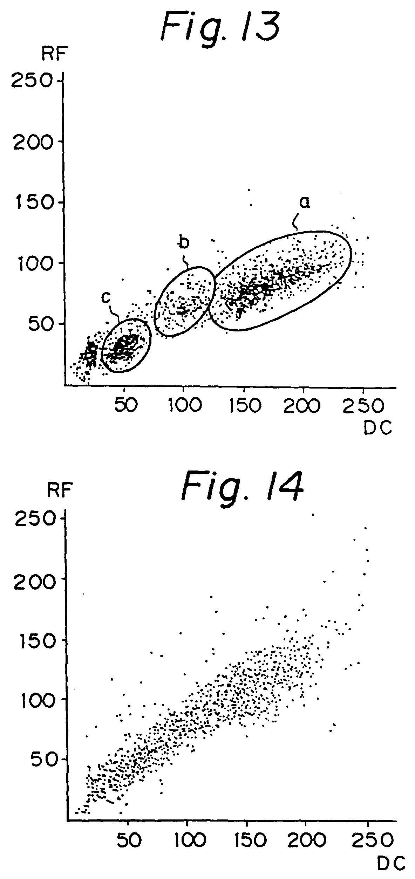

As shown above, the quaternary ammonium salt causes an undesirably high degree of damage to leukocytes and hence is not considered to be a preferred lysing agent for the purpose of leukocyte classification. Fig. 14 shows the results of a leukocyte measurement by the RF and DC methods using a quaternary ammonium salt. Obviously, the leukocytes are not separated into a plurality of populations. This is because the leukocytes are damaged so extensively by the quaternary ammonium salt that they lose almost all of the cytoplasm, though their cell membrane is left intact; as a result, the difference in the intensity of signals detected from various leukocyte species is not so great as to be clearly differentiated by the RF and DC methods to produce separate fractions on a two-dimensional distribution diagram.

Thus, it is entirely meaningless to use a quaternary ammonium salt as a cytolytic agent in a method of classifying leukocytes by the combination of the RF and DC methods. On the other hand, saponin acts only mildly on leukocytes and suffers the already-described disadvantage that it takes a prolonged time to complete preliminary treatments. Furthermore, the ability of saponin to lyse blood is not consistent.

The surfactants of the first and second groups to be used in the present invention do not damage leukocytes as violently as quaternay ammonium salts but act more strongly on leukocytes than saponin and will damage the individual leukocytes to an extent that is very favorable for the purpose of leukocyte classification.

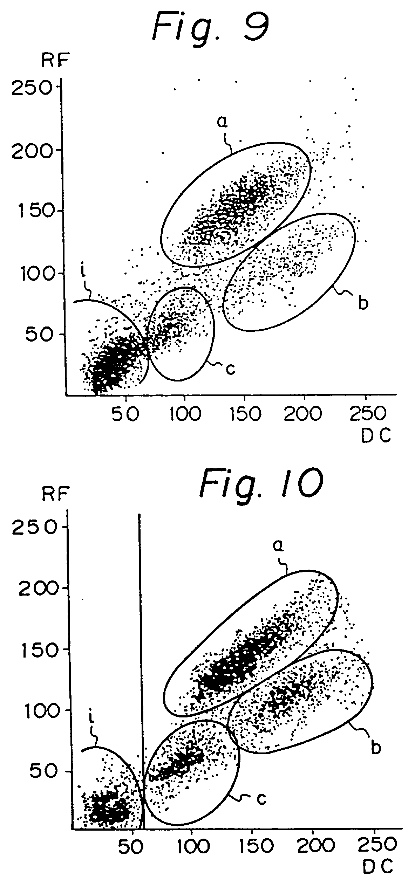

Fig. 9 shows the results of fractionating leukocytes by performing RF and DC measurements on a blood sample before addition of a cytolytic agent. Obviously, granulocytes a are clearly separated from monocytes b but lymphocytes c and erythrocytes i are not. Furthermore, this diagram shows the strong effects caused by simultaneous passage of erythrocytes. The described classification and counting of leukocytes are therefore impossible unless some modification is made.

However, even if no cytolytic agent is added, a sample in which only the leukocytes have been carefully separated permits the leukocytes to be fractionated to some extent through measurements by the RF and DC methods. The results of a fractionation by this approach are shown in Fig. 10.

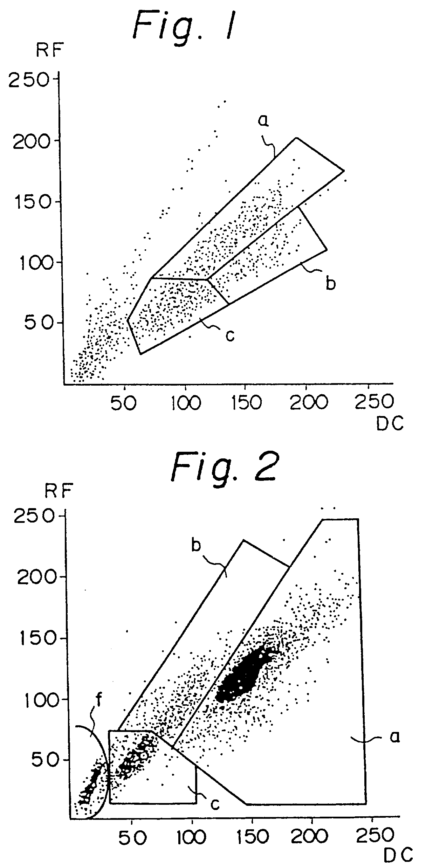

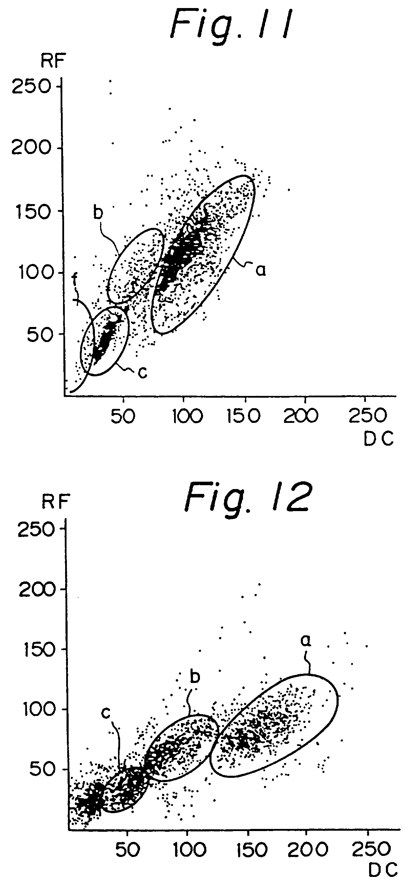

Fractions were obtained by separating the leukocytes in blood through centrifugation by identifying differences in specific gravity using Percoll-Hypaque, and the obtained fractions were subjected to measurement. Some portion of the erythrocytes is left unseparated in the sample. As Fig. 10 shows, granulocytes a, monocytes b and lymphocytes c can be separated without using a cytolytic agent. However, separating only leukocytes as in the case described above is very difficult and time-consuming even for a skilled operator, and in addition to that, this work cannot be automated. In practice, therefore, the method in which the erythrocytes are lysed to leave only leukocytes intact has to be adopted. However, if a cytolytic agent is added to lyse erythrocytes, the leukocytes are also subjected to the action of that agent. Hence, it is necessary to ensure that even after the erythrocytes have been lysed, granulocytes, monocytes and lymphocytes are sufficiently separated to come out clearly on a two-dimensional distribution diagram. Fig. 11 shows the results of an experiment in which a cytolytic agent which was the same as what is used in Example 2 to be described later in this specification was added to a blood sample which was also the same as the blood employed in the operation of separating only leukocytes, and in which the leukocytes were classified through measurements by the RF and DC methods for a period of 5 seconds following elapse of 14 seconds from the addition of the lytic agent. The scale of Fig. 11 is the same as that of Fig. 10. One can readily see that the leukocytes decreased in overall size after the addition of the lytic agent. It is also interesting to note that the speed at which monocytes decrease in volume is particularly high and that the monocytes which produced stronger DC signals than granulocytes before the addition of the lytic agent yielded weaker signals after the addition of the agent. Fig. 2 showing the results of Example 2 to be described herein is drawn on an enlarged scale compared to Fig. 11. Figs. 10 and 2 differ in scale with respect to both the DC and RF methods. Stated more specifically, after the addition of a lysing agent, the cells decrease in size, and the signals detected also become weak. In Fig. 2, the signals detected are amplified for the sake of clarity (so are the results of the examples to be described herein).

The methods of the present invention lyse erythrocytes and damage individual leukocytes by means of the reagents of the present invention and classifying and count leukocytes in accordance with the difference in speed at which the cells change in volume in dependence upon the amount of damage sustained by the leukocytes. In addition, the present invention which employs a cytolytic agent does not have to rely upon centrifugation of the type described above.

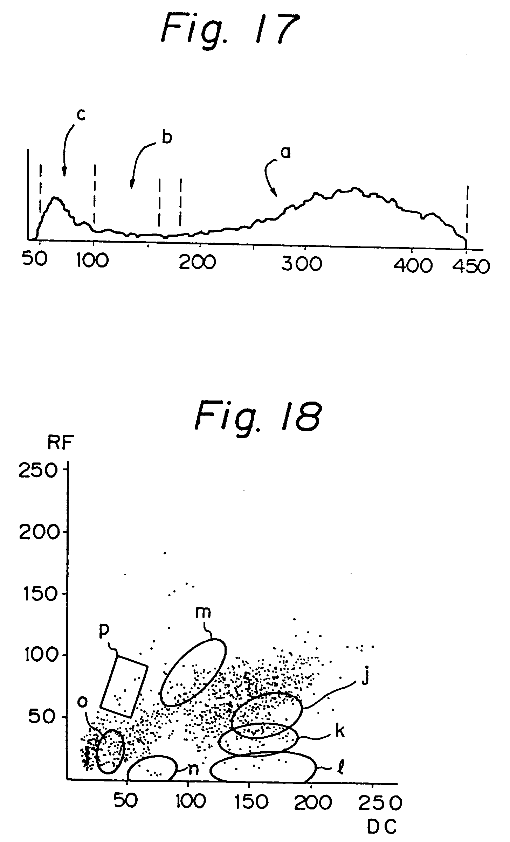

We have so far described the ability of the reagents of the present invention to enable classification and counting of normal leukocyte cells. Surprisingly enough, the present inventor has also found that if erythrocytes are lysed using the reagents of the present invention and when leukocytes are measured by the DC and RF methods, not only the normal leukocyte cells described above but also various types of abnormal cells can be classified and counted. Fig. 18 is a diagram showing the positions at which various types of abnormal cells appear. The distribution diagram shown in Fig. 18 was obtained by measuring the blood of healthy persons using the reagents of the present invention, and the dots in this diagram represent the values obtained from normal cells. If any population of abnormal cells is to be found, it will appear in the vicinity of one of the regions surrounded by the solid lines. Among the abnormal cells to be detected, the population of left-shift cells will essentially appear in region j, immature granulocytes (IG) in region k, blasts in region l, heterolymphocytes in region m, lymphoblasts in region n, nucleated erythrocytes in region o, and platelet agglutinating cells in region p. Neutrophils consist of segmented cells and band cells and the more juvenile the cells are, the less segmented their nuclei are and band nuclei will predominate. The increase in the number of juvenile cells, or band cells, in leukocytes is called a "left shift". Left-shift cells, immature granulocytes and blasts are normal leukocytes that are in the state of immaturity or juvenility, and their juvenility increases in the order written. It is generally held that the more immature the leukocytes are, the smaller their specific gravity and the smaller the intensity of signals that are produced by the RF method. Therefore, the most juvenile blasts appear in the lowest part of Fig. 18. The platelet agglutinating cells appear when platelets aggregate for some reason to glow to a size comparable to leukocytes, and it is important to differentiate and eliminate these cells if one wants to obtain correct leukocyte counts.

There is no prior art technique that has ever succeeded in classifying and counting abnormal leukocyte cells by either the DC or RF methods or combinations thereof. A method has been proposed for detecting abnormal leukocyte cells by optical principles of measurement (see Japanese Patent Public Disclosure No. 88896/1986) but as already pointed out, detection by the disclosed optical principles of measurement requires sophisticated equipment and very complicated procedures. Furthermore, according to the disclosure in this patent, the nuclei of all leukocytes except basophils become naked. Therefore, the method described in this patent is capable of detecting abnormal cells and basophils but is unable to classify and count normal leukocytes other than basophils. A need therefore arises for the provision of a separate measuring channel in order to allow for classification and counting of normal leukocyte cells. It was not until the development of the methods and reagents of the present invention that simultaneous classification and counting of both normal and abnormal leukocyte cells became possible.

Prior art techniques in which reagents containing polyoxyethylene-based surfactants are used for the purpose of leukocyte classification include the methods described in Japanese Patent Public Disclosure No. 22891/1979 and US-A-4,099,917 as well as the method described in Japanese Patent Public disclosure No. 71857/1987 (corresponding to EP-A- 214,613). All of these methods are within the scope of the method of the second category which employs a flow system as already described herein under the section of "Prior Art" and they utilize an optical apparatus for measurement. Since these methods employ a completely different principle of measurement than the methods of the present invention, needless to say the reagents disclosed in the above-mentioned patents cannot be directly applied to the methods of the present invention whose operating principle is detection of impedance. In addition, according to the experiments conducted by the present inventor, some of the polyoxyethylene-based surfactants disclosed in those prior patents are not only unsuitable for use in the methods of the present invention for leukocyte classification but also lack the ability to dissolve erythrocytes. On the contrary, certain polyoxyethylene-based surfactants are used as sheath solutions for blood cell counting apparatus that are essential for the protection of erythrocytes (see Japanese Patent Public Disclosure No. 87233/1987, and Japanese Patent Application Nos. 261386/1987 and 261387/1987).

As will be understood from the above explanation, there are many polyoxyethylene-based surfactants that have no ability to dissolve erythrocytes and those which are suitable as compositions to be incorporated in the reagents of the present invention for leukocyte classification are even more limited in number.

The present inventor performed screening on a great number of anionic and nonionic surfactants including polyoxyethylene-based surfactants and found as a result that the surfactants of the first and second groups set forth in Claim 1 are suitable for the purposes of the present invention.

The objects of the present invention can be attained if the carbon number of R₁ in the general formula set forth in Claim 1 is 10 - 22 and if n (the number of added moles) is 8 - 30. For better fractionation of leukocytes, the carbon number of R₁ is preferably 12 - 18, with n being 12 - 15.

When fresh blood that has just been sampled is measured using the reagent set forth in Claim 1 (this reagent is hereinafter referred to as a lysing reagent), lymphocytes, monocytes and granulocytes can be fractionated in a desired way. However, if blood, especially abnormal blood, is left to stand for several hours, say 8 hours, after sampling, a two-dimensional distribution as shown in Fig. 12 is obtained. Regions a, b and c in Fig. 12 denote those regions where granulocytes, monocytes and lymphocytes are respectively found when normal blood that has just been sampled is measured. An abnormal increase is observed in the number of cells in normal region b for monocytes, and lymphocytes c and monocytes b are poorly fractionated, with a decrease in the number of granulocytes a. This is because as time goes by after blood sampling, leukocyte cells in abnormal blood become brittle and highly sensitive to the action of the lysing reagent added, causing granulocytes, in particular part of the cells of neutrophils, to decrease in volume more rapidly than when they do right after blood sampling. Another reason is that the lysing reagent acts on the blood so abruptly that it will not act uniformly on individual corpuscles (the corpuscles are not dispersed uniformly in the solution of lysing reagent) but rather act strongly on some corpuscles in preference over others.

These problems could be partly solved by merely diluting the blood before the addition of the lysing reagent using a common diluting solution for achieving uniform dispersion of corpuscles. However, the present inventor figured out certain reagents that were capable of allowing leukocytes to be classified and counted in a very consistent way even when blood was left to stand for several to several tens of hours after sampling. Such reagents are those set forth in Claims 1 to 3, 7 and 8.

The reagent set forth in Claims 1 and 7 is composed of two fluids. The first fluid is a so-called liquid diluent and contains a hyperosmotic agent. The second fluid is the lysing reagent. Blood is first diluted and the corpuscles dispersed uniformly with the first fluid which is a liquid diluent. Then, the second fluid which is a lysing reagent is added to dissolve the erythrocytes and to allow the leukocytes to be classified and counted.

The hyperosmotic agent present in the first fluid adjusts the osmotic pressure of the first fluid to 285 mmol/kg (m0sm) or above. A method in which a solution for leukocyte measurement is processed into a hypertonic solution (hyperosmotic solution) is described in Japanese Patent Public Disclosure No. 71857/1987, but as already mentioned, optical principles of measurement are adopted in the invention described in this patent and the action and the effect of the hypertonic solution employed in it are entirely different from those of the first fluid which is rendered hypertonic in the present invention. According to said patent, lymphocytes become circular saw-toothed blood cells by the action of the hypertonic solution on corpuscles and this contributes to improved discrimination between detection signals for lymphocytes and noise. In the absence of any detailed description in this patent, it is not clear why the change to circular saw-toothed blood cells contributes to the improved ability to distinguish from noise, but it would be some reason that is closely related to the principles of optical measurement. According to the principles of impedance measurement employed in the present invention, information about the size or interior of cells is detected by the DC or RF method and deformations of the contour of cells will essentially result in no effect on the intensity of signals detected. The purpose of rendering hyperosmotic the first fluid in the present invention is as follows: by placing corpuscles under high osmotic pressure, the cell membranes of corpuscles are dehydrated so as to become rigid enough to be protected against the violent shock that would otherwise occur when the second fluid which is a lysing reagent is subsequently added. Besides this protecting effect, placing corpuscles under high osmotic pressure causes the shrinkage of cell membranes, thereby allowing the corpuscles to decrease in volume. As already mentioned, information on the size of cells is to be detected according to the principles of impedance detection, so if the volume of corpuscles is excessively reduced, it becomes difficult to distinguish them from noise. For this reason, placing corpuscles under high osmotic pressure has generally been avoided in leukocyte measurements according to the principles of impedance detection. Quite surprisingly, however, the present inventor found that even when the second fluid which is a lysing reagent was allowed to act after the corpuscles had been placed under high osmotic pressure by treatment with the first fluid, leukocyte signals could be distinguished from noise as clearly as when the lysing agent was allowed to act on the blood from the beginning. This would be because the cell membranes of leukocytes were protected by the action of the first fluid. In this way, the reagent composed of two fluids, the first one of which was a hyperosmotic fluid, allowed leukocyte cells to be protected from the violent shock that would be caused by the second fluid, which was a lysing reagent, and yet the reagent assured satisfactory discrimination of leukocyte signals from noise. This enabled leukocytes to be classified and counted in a consistent way without permitting neutrophils to get into the region of monocytes in the above-described two-dimensional distribution diagram even when a measurement was conducted on blood that had been left to stand for several tens of hours after sampling. Fig. 13 shows the results of an experment in which blood that was the same as what was employed to obtain the results shown in Fig. 12 was measured with the above-described two-component reagent system when it had been left to stand for 8 hours after sampling as in the case shown in Fig. 12. Obviously, granulocytes a, monocytes b and lymphocytes c were clearly fractionated without any abnormal increase in the number of cells in the region of monocytes. It should be mentioned here that the osmotic pressure of the first fluid must be at least about 285 mmol/kg (m0sm). Below this value, the membranes of leukocytes will not be fully protected. Hyperosmotic agents that can be used with advantage include ethylene glycols and alcohols.

Ideally, the reagent of the present invention is composed of two fluids, with the first fluid being made a hyperosmotic solution. On the other hand, the two-component system is not desirable since its use complicates the procedures of analysis or the composition of analyzing equipment. Therefore, a one-component reagent system in which the lysing reagent is formulated as a hyperosmotic fluid has been figured out (this is the reagent set forth in Claim 6). This reagent is not capable of protecting the membranes of corpuscles as completely as the two-component system but the present inventor confirmed that even this one-component reagent system had the capability to allow leukocytes to be classified and counted consistently at practically satisfactory levels without permitting neutrophils to get into the region of monocytes in the above-described two-dimensional distribution diagram even when a measurement was conducted on blood that had been left to stand for several tens of hours after sampling.

Whether the reagent is of a two-component or one-component system, it is more effective for a fixing agent to be incorporated in the hyperosmotic fluid. This fixing agent serves to assist in the leukocyte cell membrane protecting action of the hyperosmotic fluid. Prior art techniques that perform leukocyte classification using reagents that contain a fixing agent are described not only in the aforementioned Japanese Patent Public Disclosure Nos. 22891/1979 and 71857/1987 and National Publication of Translated Version No. 502277/1986 but also in US-A-3,741,875.

In all of these prior art techniques, the solution has to be heat-treated after the addition of a fixing agent. In contrast, there is no need to perform a heat treatment even after the addition of the reagent containing a fixing agent. In all of the prior art techniques mentioned above, the fixing agent is responsible for the important part of the action to be displayed by the reaction reagent, so there is a particular need to promote the reaction of the fixing agent by performing a heat treatment. On the other hand, the fixing agent in the reagent of the present invention plays only an auxiliary role and there is no particular need to promote the reaction by performing a heat treatment. This absence of the need for heat treatment offers a great benefit which can be enjoyed by simplifying the method or apparatus of analysis.

In the present invention, at least one solubilizing agent selected from among the five groups listed in Claim 5. may be incorporated and this is also effective for the purpose of improving the precision of leukocyte classification. Solubilizing agents generally serve to dissolve slightly water-soluble or completely water-insoluble substances to an extent that apparently exceeds their solubility and are extensively used in industrial and biological fields. In medical fields, solubilizing agents are used to make water-soluble drugs from vitamins and hormones. However, the solubilizing agents employed in the present invention have activities that are different from the general functions described above. Monocytes in leukocytes have inherently strong reactivity with lysing reagents containing surfactants and they are the cells that decrease in volume most rapidly after the addition of lysing reagents. However, the present inventor found that when a solubilizing agent was incorporated in the lysing reagent or in the liquid diluent that was to be used before the lysing reagent, the solubilizing agent promoted the action of the lysing reagent on monocytes, thereby causing them to decrease in volume even more rapidly. In other words, the solubilizing agents used in the present invention act selectively on monocytes. It was also found that these solubilizing agents which acted selectively on monocytes were effective not only for the reagents characteristic of the present invention that contained a surfactant which was selected from the group consisting of the aforementioned surfactants of the first and second groups, but also for all reagents for leukocyte classification that could be learned from the prior art. Among these solubilizing agents, those of the second group set forth in Claim 5 are particularly effective.

A surfactant can also be incorporated in the first fluid (the liquid diluent rendered hyperosmotic) in the already-described two-component reagent system (as in the reagent set forth in claims 2 and 8 The surfactant to be used in this case may be selected from among the surfactants of the first and second groups set forth in claims 1 and 7 or alternatively they may be common surfactants. The principal activity of the surfactants to be contained in the first fluid is not to dissolve corpuscles but is closer to the activity of the solubilizing agents described above. In other words, the osmotic pressure of the first fluid is high enough to weaken the lysing action of the surfactants in it and they would serve to perform a preliminary treatment for selectively promoting the shrinkage of monocytes among the corpuscles which are to be lysed as a result of the subsequent addition of the lysing reagent. It is therefore required that the surfactant in the second fluid has a substantially stronger cytolytic activity than the surfactant in the first fluid. Otherwise, the dissolving reaction of corpuscle cells will not proceed even if the second fluid is added. If the first fluid has a higher osmotic pressure than the second fluid, the condition noted above is satisfied even if the surfactants contained in the first and second fluids are of the same kind and have the same concentration.

All of the reagents described above of the present invention contain a buffer agent for adjusting the solution to a predetermined pH. At pHs below the predetermined value, the lysing reaction is retarded and the state where the leukocytes can be classified will not be reached within the requisite time for practical purposes. At pHs above the predetermined value, the lysing reaction is so greatly accelerated that it becomes difficult to classify the leukocytes in a stable state.

Although not characteristic of the present invention, common antioxidants or electroconductive adjusting agents may be incorporated, as required, in all of the reagents of the present invention.

The attached drawings may be explained as follows.

Fig. 1 is a two-dimensional distribution diagram showing the results of classification of leukocytes in Example 1; the horizontal axis of this two-dimensional distribution diagram plots the relative intensity of signals as obtained when the measurement was conducted by the DC method and the vertical axis plots the relative intensity of signals as obtained when the measurement was conducted by the RF method; the dots in Fig. 1 represent the cells that produced DC and RF signals the intensities of which are respectively associated with the DC and RF methods; and DC and RF in Fig. 1 represent the intensities of DC and RF signals, respectively.

Fig. 2 is a two-dimensional distribution diagram showing the results of classification of leukocytes in Example 2; for the description of this diagram, see the relevant explanation of Fig. 1.

Fig. 3 is a two-dimensional distribution diagram showing the results of leukocyte classification conducted as in the case of Fig. 2 except that the blood sample was left to stand for 90 seconds after the addition of a lysing agent.

Fig. 4 is a graph showing the results of leukocyte classification conducted by the DC method alone in Example 2 under the same conditions as those employed in obtaining the data shown in Fig. 3.

Fig. 5 is a two-dimensional distribution diagram showing the results of leukocyte classification conducted in Example 4 by the combination of the RF and DC methods using a reagent for the measurement of basophils.

Fig. 6 is a graph showing the results of leukocyte classification conducted by the DC method alone in Example 4.

Fig. 7 is a two-dimensional distribution diagram showing the results of leukocyte classification conducted in Example 5.

Fig. 8 is a two-dimensional distribution diagram showing the results of leukocyte classification conducted in Example 6.

Fig. 9 is a two-dimensional distribution diagram obtained when a blood sample diluted in the absence of a cytolytic agent was measured by the RF and DC methods.

Fig. 10 is a two-dimensional distribution diagram obtained when blood in which only leukocytes were separated in the absence of a cytolytic agent was measured by the RF and DC methods.

Fig. 11 is a two-dimensional distribution diagram obtained when a blood sample containing the same cytolytic agent as that used in Example 2 was measured by the RF and DC methods; the scale of Fig. 11 is the same as that of Figs. 9 and 10; and the scale of Fig. 2 is enlarged as compared to Fig. 11.

Fig. 12 is a two-dimensional distribution diagram obtained when a blood sample that had been left to stand for 8 hours after sampling and to which a reagent containing no hyperosmotic agent was added was measured by the RF and DC methods.

Fig. 13 is a two-dimensional distribution diagram obtained when a blood sample that had been left to stand for 8 hours after smapling and to which a reagent containing a hyperosmotic agent was added was measured by the RF and DC methods.

Fig. 14 is a two-dimensional distribution diagram showing the results of leukocyte classification conducted by the RF and DC methods using a quaternary ammonium salt as a cytolytic agent.

Fig. 15 is a cummulative size-frequency distribution curve enabling one to confirm that the magnitude of signals from leukocytes is sufficiently large as compared to the magnitude of signals from erythrocyte membranes (ghosts) and noise; the horizontal axis of the graph in the diagram plots the signal threshold values of an automatic blood cell counter, and the vertical axis plots the number of detected signals exceeding a certain signal threshold value; in Fig. 15, A and B signify flat portions of the cumulative size-frequency distribution curve.

Fig. 16 is a size-frequency distribution curve constructed by plotting the number of leukocytes for each threshold level as calculated from the cumulative size-frequency distribution curve in Fig. 15; in this diagram, C signifies the population of erythrocyte ghosts and noise, D is the first population of leukocytes, and E is the second population of leukocytes.

Fig. 17 is the leukocyte size-frequency distribution curve obtained by performing the DC method as described in an example of US-A- 4,485,175.

Fig. 18 is a diagram showing the positions at which various types of abnormal cells appear in a two-dimensional distribution diagram.

Fig. 19 is a two-dimensional distribution diagram as obtained in the measurement of blood from a patient with acute lymphocytic leukemia (ALL).

Fig. 20 is a two-dimensional distribution diagram as obtained in the measurement of blood from a patient with adult T-cell leukemia (ATL).

Figs. 21 and 22 are two-dimensional distribution diagrams as obtained in the measurement of blood from a patient with acute myelocytic leukemia (AML).

Fig. 23 is a two-dimensional distribution diagram for the case where left shifts j and heterolymphocytes m appeared in one specimen.

Figs. 24 and 25 are two-dimensional distribution diagrams for the case where immature granulocytes k appeared.

In Figs. 1 to 14, symbols a to i have the following meanings:

| | |
|---|---|
| a: granulocytes | b: monocytes |
| c: lymphocytes | d: eosinophils |
| e: basophils | f: erythrocyte ghosts |
| g: leukocytes other than eosinophils | |
| h: leukocytes other than basophils | |
| i: erythrocytes. | |

In Figs. 18 to 25, symbols j to p have the following meanings:

| | |
|---|---|
| j: left shifts | |
| k: immature granulocytes (IG) | |
| l: blasts | m: heterolymphocytes |
| n: lymphoblasts | o: nucleated erythrocytes |
| p: platelet aggregation | |

For the description of RF and DC in Figs. 2 - 14 and 18 - 25, see the relevant explanation of Fig. 1.

### Example 1

The following is an example in which Sandet EN [the trade name of Sanyo Chemical Industries, Ltd. for the chemical formula: C₁₂H₂₅-O-(CH₂CH₂O)₂SO₃Na], one kind of surfactant from among those in the first group, was used as a cytolytic agent.

Blood (60 µl) was diluted with 5 ml of a 0.125% solution of said cytolytic agent and 10 ml of a liquid diluent consisting of 1/60 M phosphate buffer agent and 0.6% sodium chloride, and measurement was started 14 seconds after the addition of the cytolytic agent under the following conditions: pH, 7.0; osmotic pressure, 120 mmol/kg (m0sm); and solution temperature, 26°C. The results of leukocyte measurement conducted for 5 seconds are shown in Fig. 1. The horizontal axis of the two-dimensional distribution diagram shown in Fig. 1 plots the relative intensity of signals as obtained when the measurement was conducted by the DC method, and the vertical axis of Fig. 1 plots the relative intensity of signals as obtained when the measurement was conducted by the RF method. The dots in Fig. 1 represent cells that produced DC and RF signals the intensities of which are respectively associated with the DC and RF methods.

As Fig. 1 shows, the leukocytes were fractionated into populations that could be presumed to consist of lymphocytes, monocytes and granulocytes, respectively. Since the measurement was conducted by a combination of the RF and DC methods to construct a two-dimensional distribution diagram, the leukocytes could be fractionated more clearly than when they were classified into three types by using the DC method alone, as in the prior art, the results of which are shown in Fig. 17.

### Example 2

Measurements were conducted under the same conditions as those employed in Example 1 except that 4.0% conc. Emulmin 140 [the trade name of Sanyo Chemical Industries, Ltd.; containing 56% C₁₈H₃₇-O-(CH₂CH₂O)₁₄H, 34% C₁₆H₃₃-O-(CH₂CH₂O)₁₄H, and 7% C₁₄H₂₉-O-(CH₂CH₂O)₁₄H] and 0.5% conc. Emulgen 420 [the trade name of Kao Corp. for C₁₈H₃₇-O-(CH₂CH₂O)₁₃H] were used. The results are shown in Fig. 2.

As in Example 1, the leukocytes were classified into three populations. The fact that these populations consisted of lymphocytes, monocytes and granulocytes, respectively, was confirmed by analyzing the samples containing the individually separated leukocyte species and by performing a correlation test with the visual counting method.

Comparing Fig. 2 with Fig. 1, one will be able to see that the position of the appearance of monocytes relative to granulocytes is different. This is because the speed at which the individual leukocytes change in volume differs depending upon the kind and concentration of lysing agent. In Fig. 2, monocytes which are inherently larger than granulocytes appear at smaller positions (smaller intensities of DC signals) than granulocytes because the monocytes were damaged at a notably higher speed than the other leukocytes.

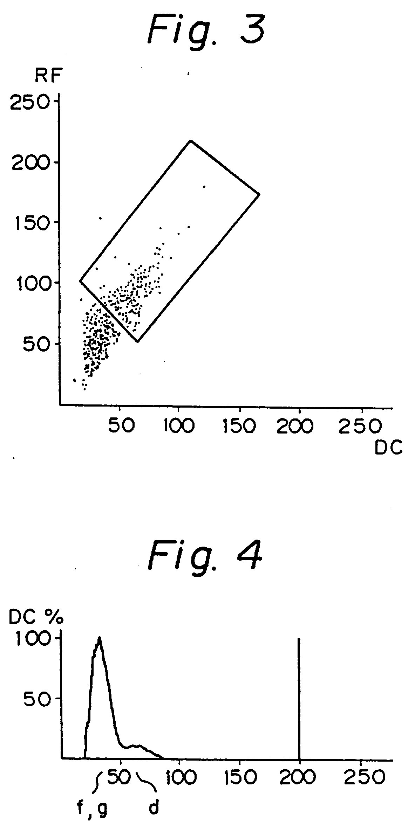

Except for the use of the cytolytic agent described above, a blood-containing liquid diluent was prepared in the same manner as in Example 1, and this sample was left to stand for 90 seconds after the addition of the lysing agent. A measurement was conducted during the subsequent 5 seconds. The results are shown in Fig. 3. The present inventor found in this measurement that only eosinophils were left intact and could be selectively detected as separate entities from the other leukocytes. This could be explained by the high resistance of the membranes of eosinophils against the lysing agent employed. When the measurement was conducted by the DC method alone under the same conditions, the results shown in Fig. 4 were obtained, from which one can see that eosinophils were satisfactorily separated from the other leukocytes.

### Example 3

A measurement was conducted for 5 seconds following the passage of 13 seconds after the addition of the lysing agent under the same conditions as those employed in Example 2, except that the pH was adjusted to 10.0. In this case, too, eosinophils were successfully separated from the other leukocytes as in Example 2.

In the three examples shown above, the concentration of the lysing agents was adjusted to 0.125% or 4.5%. Similar results will be obtained if the concentration of lysing agents is within the range of 0.01 - 15%, preferably 0.05 - 10%. The pH was adjusted to 7.0 or 10.0 but satisfactory results can be obtained if it is within the range of 4.0 - 12.0, preferably 7.0 - 10.0. The osmotic pressure was held at 120 mmol/kg (m0sm), but good results can be obtained if it is within the range of 50 - 400 mmol/kg (m0sm), preferably 100 - 200 mmol/kg (m0sm). The solution temperature was 26°C but equally good results can be obtained in the measurement if it is within the temperature range of 10 - 37°C. It should, however, be noted that the time at which a measurement is started following the addition of lysing agents must be adjusted depending upon the temperature.

The composition of the liquid diluent containing a buffer and other ingredients, as well as the concentrations of such ingredients, are in no way limited to the examples shown above. In the above examples, the cytolytic agent and the liquid diluent were prepared as separate solutions, but they may be preliminarily mixed to prepare a single lysing fluid.

### Example 4

In the three examples described above, basophils in the granulocytes were not separated. In order to separate and count basophils, the following procedures may be taken.

Blood (80 µl) was diluted with 10 ml of a reagent for basophil measurement containing 1.44% conc. Emulgen 123-P [the trade name of Kao Corp. for the chemical formula: (C₁₂H₂₅-O-(CH₂CH₂O)₂₃H], 0.435% conc. potassium o-phthalate, 0.025% conc. hydrochloric acid and 0.025% conc. nitric acid, and measurement was conducted for 5 seconds following the lapse of 13 seconds after the addition of the lysing agent under the following conditions; pH, 3.0; osmotic pressure, 60 mmol/kg (m0sm); and solution temperature, 33°C.

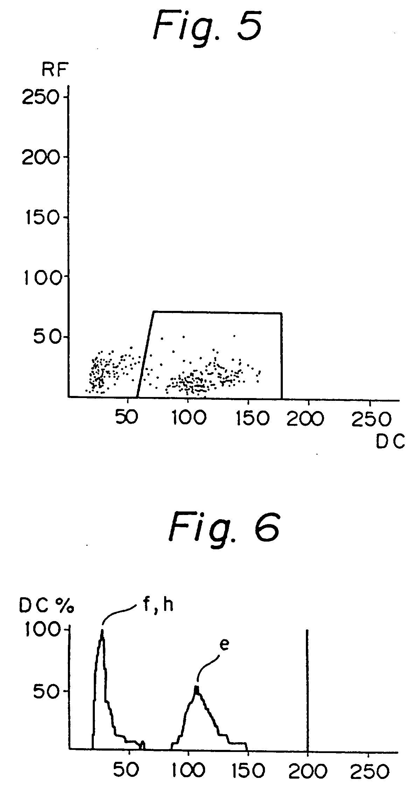

The results of measurement conducted by the combination of the RF and DC methods are shown in Fig. 5, and the results of measurement conducted by the DC method alone are shown in Fig. 6. In either case, basophils were selectively left intact and could be measured as separate entities from the other leukocytes. This is because the lysing agent used caused the nuclei of all leukocytes other than basophils to become nacked, leaving only basophils intact.

Surfactants that can be used in the reagent for basophil measurement described above are not limited to Emulgen 123-P and may be selected from among any nonionic surfactants that are represented by the general formula: R-O-(CH₂CH₂O)ₙH, provided that R is an alkyl group having 10 - 20 (preferably 12 - 18) carbon atoms, and n is 6 - 100 (preferably 15 - 40). Hydrochloric acid and nitric acid are used to adjust the pH to about 3.0 and at least one acid may be selected from the group consisting of hydrochloric acid, nitric acid and acetic acid. The osmotic pressure may be of any value within the range of 10 - 400 (preferably 20 - 100). The solution temperature may be any value within the range of 10 - 40°C.

While the basophils are classified and counted as described above, lymphocytes, monocytes, granulocytes and eosinophils in the granulocytes are classified and counted by the method employed in Example 3. The number of neutrophils is calculated by substracting the numbers of eosinophils and basophils from the total granulocyte count. In this way, the numbers and percentages of lymphocytes, monocytes, neutrophils, eosinophils and basophils in all the leukocytes of interest can be determined.

Similar measurements were conducted on 105 specimens of blood by the methods described above and the results were compared with the performance of the visual counting method by which 500 cells were observed per specimen. The correlation coefficients for the percentages of five leukocyte types are shown in Table 2.

**Table 2**

| | |
|---|---|
| Lymphocytes | 0.95 |
| Monocytes | 0.43 |
| Neutrophils | 0.90 |
| Eosinophils | 0.95 |
| Basophils | 0.85 |

As the above data shows, the method employed in Example 4 showed good correlation with the visual counting method which is the standard technique currently employed for classifying and counting leukocytes, and it will be understood that the method of the present invention is capable of clearly classifying the leukocytes into five types and counting their respective numbers. The results obtained in Example 4 are dramatic in that they show the first success in classifying and differentiating the leukocytes into five types by a method which depends on electrical detection of the change in impedance that occurs when corpuscles pass through an orifice.

### Example 5

A composition of the two-component reagent system of the present invention and the results of a measurement conducted using said reagent are described below.

### Composition of reagent

| First fluid (liquid diluent) | |
|---|---|
| Buffer agents: | |
| Disodium phosphate (duodecahydrate) Na₂HPO₄·12H₂O | 9.0 g |
| Monosodium phosphate (anhydrous) NaH₂PO₄ | 3.0 g |
| Antioxidant : | |
| EDTA-2K | 0.1 g |
| Electroconductivity adjusting agent: | |
| Sodium chloride (NaCl) | 0.16 g |
| Solubilizing agent: | |
| CHAPS | 0.4 g |
| Hyperosmotic agent: | |
| Ethylene glycol | 70.0 g |
| Fixing agent: | |
| Formalin | 50.0 g |
| Water (pH, 7.2; osmotic pressure, 1400 mmol/kg (m0sm)) | 1000 ml |

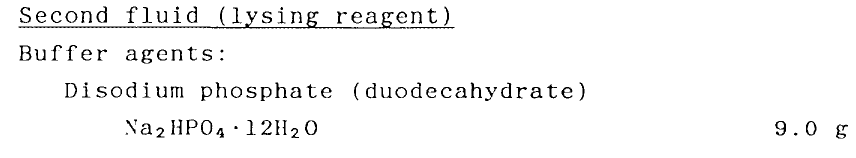

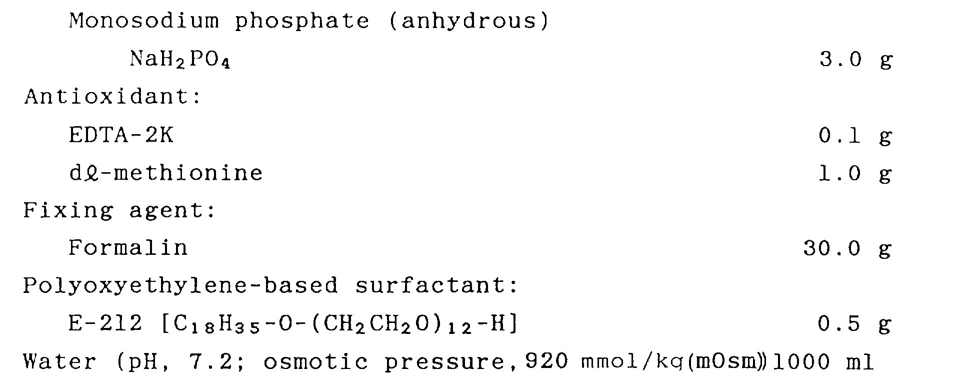

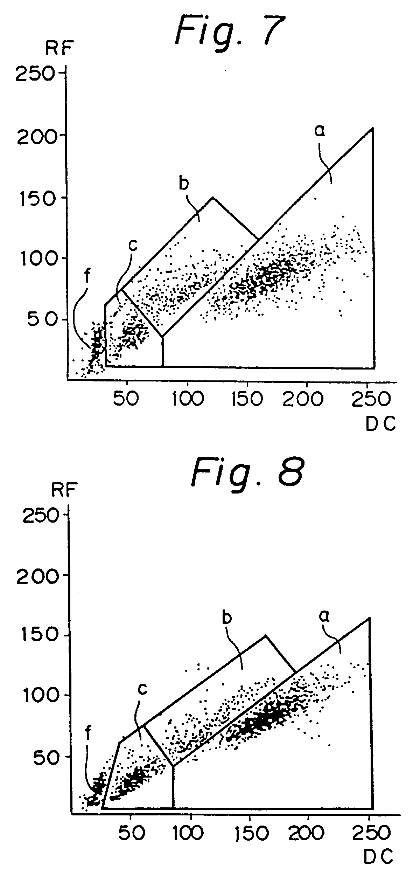

Blood (12 µl) that had been left to stand for 4 hours after sampling was mixed with 2 ml of the first fluid having a solution temperature of 26°C, and the mixture was left to stand for 25 seconds. Thereafter, 1 ml of the second fluid having a solution temperature of 26°C was added to make a 250-fold diluted sample. A measurement was conducted for 6 seconds following the lapse of 60 seconds after the addition of the second fluid. The results are shown in Fig. 7. The leukocytes were classified into three types, granulocytes a, monocytes b and lymphocytes c.

### Example 6

Another composition of the two-component reagent system of the present invention and the results of a measurement conducted using said reagent are described below.

### Composition of reagent

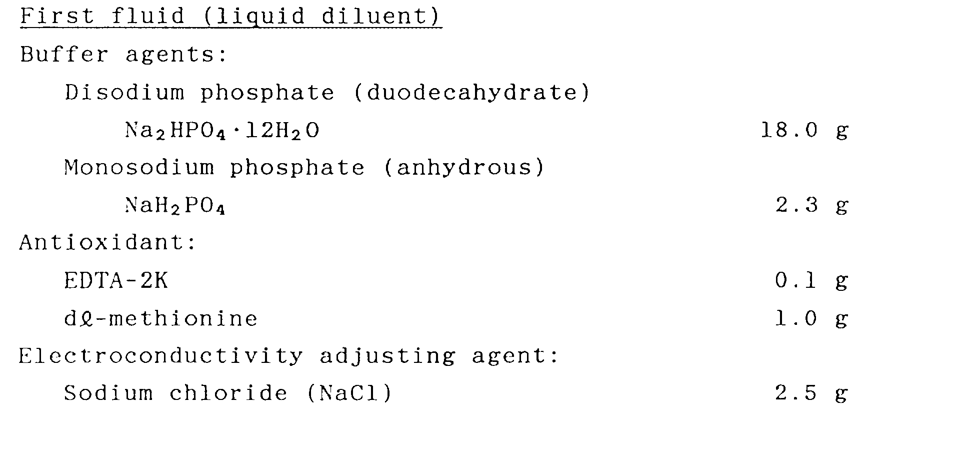

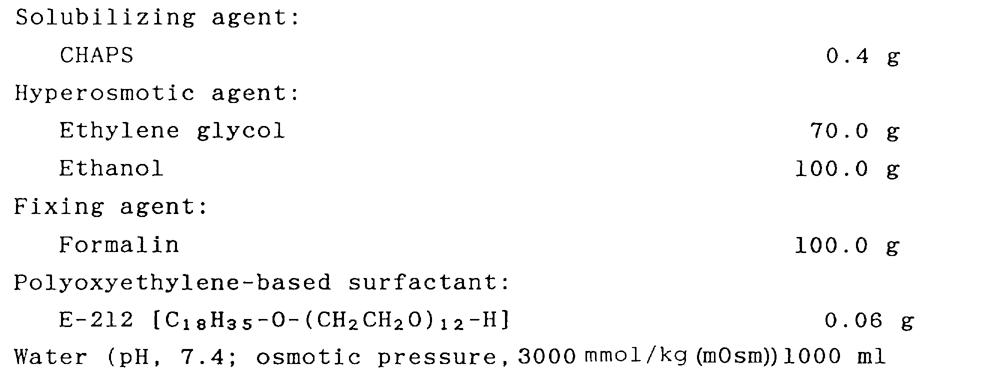

| Second fluid (lysing reagent) | |
|---|---|
| Buffer agents: | |
| Disodium phosphate (duodecahydrate) Na₂HPO₄·12H₂O | 14.0 g |
| Monosodium phosphate (anhydrous) NaH₂PO₄ | 1.5 g |
| Antioxidant : | |
| EDTA-2K | 0.1 g |
| dℓ-methionine | 1.0 g |
| Electroconductivity adjusting agent: | |
| Sodium chloride (NaCl) | 2.3 g |
| Fixing agent: | |
| Formalin | 100.0 g |
| Polyoxyethylene-based surfactant: | |
| E-212 [C₁₈H₃₅-O-(CH₂CH₂O)₁₂-H] | 0.06 g |
| Water (pH, 7.2; osmotic pressure, 1380 mmol/kg (m0sm)) | 1000 ml |

Blood (12 µl) that had been left to stand for 4 hours after sampling was mixed with 2 ml of the first fluid having a solution temperature of 26°C, and the mixture was left to stand for 20 seconds. Thereafter, 1 ml of the second fluid having a solution temperature of 26°C was added to make a 250-fold diluted sample. A measurement was conducted for 6 seconds following the lapse of 40 seconds after the addition of the second fluid. The results are shown in Fig. 8. The leukocytes were classified into three types, granulocytes a, monocytes b and lymphocytes c.

While the leukocytes were classified into three types by the method described in Example 6, the eosinophil and basophil counts were obtained by the methods described in Examples 3 and 4, respectively. The overall results were compared with those obtained for 50 specimens of blood by the visual counting method as in Example 2. The correlation coefficients determined from this comparison are shown in Table 3.

**Table 3**

| | |
|---|---|
| Lymphocytes | 0.98 |
| Monocytes | 0.83 |
| Neutrophils | 0.95 |
| Eosinophils | 0.95 |
| Basophils | 0.81 |

The data in Table 3, as compared to that in Table 2, shows a significant improvement in the correlation coefficient for monocytes.

In Examples 5 and 6, the pH was adjusted to 7.2 and 7.4, respectively, but it should be understood that pHs in the range of 6 - 8 will suffice.

### Example 7

The results of detections of abnormal leukocyte cells using the reagent described in Example 2 are shown below.

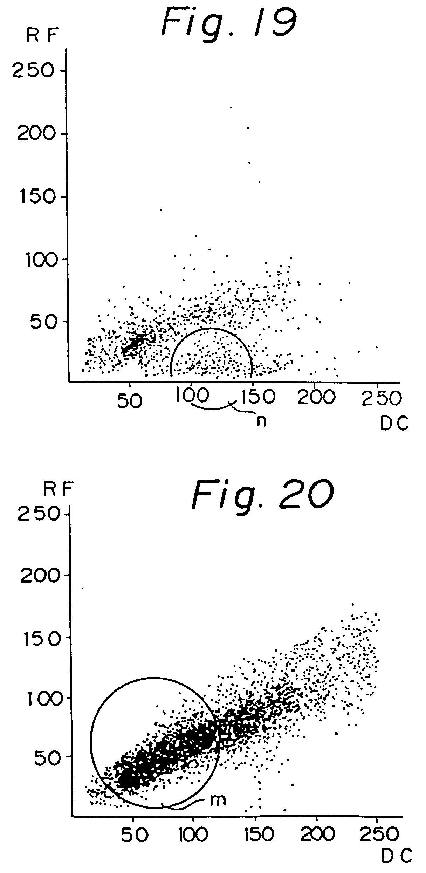

Fig. 19 shows the results of a measurement of blood from a patient with acute lymphocytic leukemia (ALL). Obviously, lymphoblasts n appeared. Detecting the appearance of lymphoblasts allows one to discover the existence of lymphocytic leukemia.

Fig. 20 shows the results of a measurement of blood from a patient with adult T-cell leukemia (ATL). Obviously, heterolymphocytes m appeared. Detection of the appearance of heterolymphocytes might well lead one to suspect the existence of a lymphocytic disease.

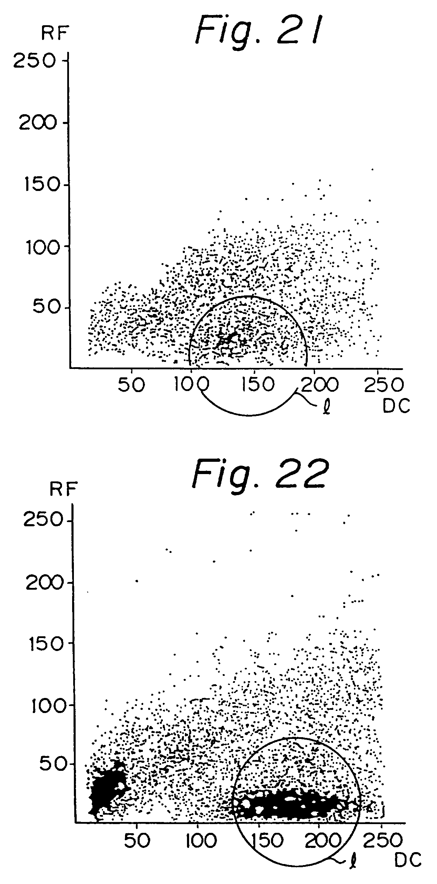

Fig. 21 shows the results of a measurement of blood from a patient with acute myelocytic leukemia (AML). Obviously, blasts 1 appeared. Detecting the appearance of blasts allows one to discover the existence of myelocytic leukemia.

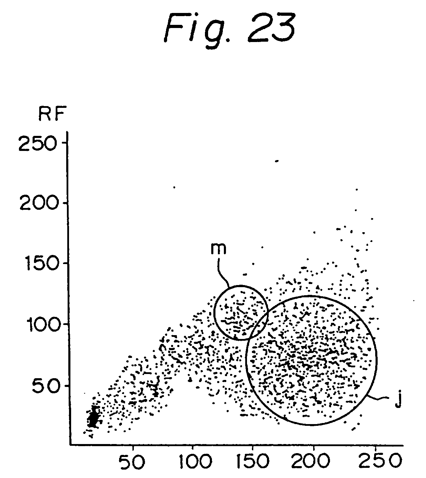

Fig. 22 also shows the results of a measurement of blood from a patient with acute myelocytic leukemia (AML). The appearance of blasts 1 is marked.

Fig. 23 shows a case where left shifts j and heterolymphocytes m appeared in one specimen.

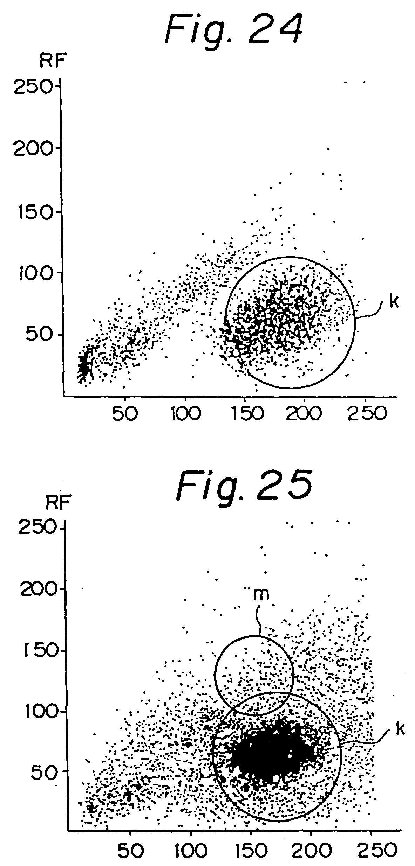

Fig. 24 shows a case of the appearance of immature granulocytes k.

Fig. 25 shows a typical case of the appearance of immature granulocytes k and heterolymphocytes m.

Various layouts can be adopted for implementing the methods of the present invention in practical applications using specific analyzers.

The simplest way that can be conceived of is as follows: three detecting portions are provided, the first one having a channel for measurement by the RF method (to be hereinafter referred to as a RF channel) and a channel for measurement by the DC method (to be hereinafter referred to as a DC channel), the second one having only a DC channel, and the third one also having only a DC channel; in the first detecting portion, the leukocytes are classified into lymphocytes, monocytes and granulocytes and their respective numbers are counted using a reagent for leukocyte classification; in the second detecting portion, the number of basophils is counted using a reagent for basophil measurement; and in the third detecting portion, only eosinophils are left intact using a reagent for leukocyte classification and the number of the eosinophils is counted so as to determine the neutrophil count by calculation. However, in this method, a different sample for measurement must be prepared for each of the three detecting portions and a need therefore arises for providing different cytolytic agents or liquid diluents, as well as storage tanks and other necessary accessories; this inevitably leads to the use of a complicated and bulky apparatus.

This defect could be eliminated by using the lysing agent described in Example 2. A method adopting this approach will proceed as follows: two detecting portions are provided, the first one having RF and DC channels, and the second one having only a DC channel; in the first detecting portion, the leukocytes are classified into lymphocytes, monocytes and granulocytes and their respective numbers are counted in the manner described above; after the lapse of a predetermined period of time when only eosinophils are selectively left intact, their number is counted; in the second detecting portion, only the number of basophils is counted in the manner described above. This method offers the advantage that the overall composition of the equipment is much simplified since only two kinds of cytolytic agent need be employed and there is no need to provide more than two detecting portions. However, with this method, the operator has to wait for a certain period of time until the eosinophils reach the state where they can be counted, so the method is not preferred for use with an automatic analyzer which is required to perform continuous measurement of many specimens in a short period of time.

This problem could be solved by providing the third detecting portion having a DC channel as in the first method. After classifying the leukocytes into three types in the first detecting portion, the spent lysing fluid is transferred to the third detecting portion, where the number of eosinophils is counted after the lapse of a predetermined standby period. In the meantime, a lysing fluid for measuring the next specimen is prepared in the first detecting portion and the measurement for classification into three types is conducted simultaneously with the counting operation in the third detecting portion. In this way, a number of specimens can be measured on a continuous basis without any interruption.

In a case where reduction in the processing time per specimen is not an important factor, the third detecting portion for measuring eosinophils may be dispensed with and the following scheme may be adopted: after the counting of the number of basophils is completed in the second detecting portion, the sample solution is withdrawn and the lysing fluid on which the measurement for classification into three types has been completed in the first detecting portion is transferred to the second detecting portion having a DC channel, in which eosinophil counts are obtained after the lapse of a certain standby period.

If abnormal leukocyte cells appear, they are detected in the detecting portion for classification into three types in any of the apparatus layouts described above.

Although not shown here, various other apparatus layouts may be readily conceived by those skilled in the art.

As described on the foregoing pages, the reagents and methods for leukocyte classification of the present invention are adapted for the purpose of classifying leukocytes into three to five types and for allowing normal and abnormal leukocytes to be classified and counted simultaneously.

## Claims

1. A reagent for classifying leukocytes in a blood sample that lyses erythrocytes and which acts on leukocytes to enable the classification and counting of leukocytes, said reagent being composed of:
(a) a first fluid which is an agent for diluting blood and which contains a hyperosmotic agent;
(b) a second fluid that contains a surfactant and which is to be added to a sample of blood that has been diluted with the first fluid, said surfactant being selected from the group consisting of:
(i) a surfactant of a first group which is a polyoxyethylene-based anionic surfactant represented by the formula:
R₁-R₂-(CH₂CH₂O)ₙ-X
where R₁ is an alkyl, alkenyl, or alkynyl group having 10-22 carbon atoms; 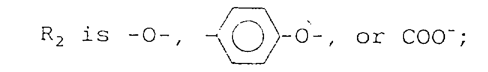 n is an integer of 8-30
X is -SO₃Na, COONa, OSO₃Na or ONa; and
(ii) a surfactant of a second group which is a polyoxyethylene-based nonionic surfactant represented by the formula:
R₁-R₂-(CH₂CH₂O)ₙ-H
where R₁ is an alkyl, alkenyl, or alkynyl group having 10-22 carbon atoms; 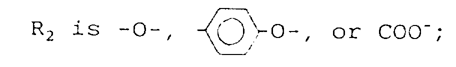 n is an integer of 8-30;
the first or second fluid further containing a buffer agent.

2. The reagent as set forth in claim 1 which also contains a surfactant in the first fluid.

3. The reagent as set forth in claim 1 in which said first or second fluid contains a solubilizing agent that selectively reduces the size of monocytes in leukocytes.

4. A reagent for classifying leukocytes in a blood sample that lyses erythrocytes and which acts on leukocytes to enable the classification and counting of leukocytes, said reagent consisting essentially of a buffer agent,
a solubilizing agent that selectively reduces the size of monocytes in leukocytes and at least one surfactant as defined in claim 1.

5. The reagent set forth in claim 3 or 4 wherein the contained solubilizing agent is at least one member selected from the group consisting of the following:
urea
thiourea
1,1-dimethylurea
ethyleneurea
methylurethane
1,3-dimethylurea
urethane (H₂NCOOC₂H₅)
N-octyl β-D-glucoside
CHAPS (3-[(3-cholamidopropyl)dimethylammonio]-1-propanesulfonate)
CHAPSO (3-[(3-cholamidopropyl)dimethylammonio]-2-hydroxy-1-propanesulfonate)
MEGA 8,9,10 (octanoyl-, nonanoyl- or decanoyl-N-methylglucamide)
sucrose monocaprate
N-formylmethylleucylalanine
guanidine thiocyanate
guanylguanidine
guanidine hydrochloride
guanidine rhodanate
guanidine nitrate
1,1,3,3-tetraguanidine
guanidine carbonate
guanidine phosphate
guanidine sulfate
sodium deoxycholate
taurocholic acid
cholic acid
sodium trichloroacetate
sodium tribromoacetate
sodium dichloroacetate
sodium dibromoacetate
sodium monochloroacetate
sodium monobromoacetate.

6. The reagent as set forth in claim 4 which contains a hyperosmotic agent.

7. A reagent for classifying leukocytes which is composed of the following two fluids (a) and (b):
(a) a first fluid as defined in claim 1; and
(b) a second fluid that contains the reagent as set forth in any one of claims 4 to 6 and which is to be added to a blood sample that has been diluted with the first fluid.

8. The reagent as set forth in claim 7 which also contains a surfactant in the first fluid.

9. A method of classifying leukocytes into lymphocytes, monocytes and granulocytes by (a) detecting the change in electric impedance at high frequency RF method) or by (b) detecting the change in current due to differences in conductivity between suspended particles and a fluid medium in which they are suspended . (DC method) using the reagent as set forth in any one of claims 1 to 8.

10. A method of classifying leukocytes into four types, lymphocytes, monocytes, eosinophils and granulocytes other than eosinophils, which comprises
(a) classifying the leukocytes in a blood sample into three types, lymphocytes, monocytes and granulocytes, by the method as set forth in claim 9, and
(b) counting by both the RF and DC methods or by the DC method alone the number of eosinophils that selectively remain intact after the passage of a predetermined period of time in the sample that has been used for Step (a).

11. A method of classifying leukocytes into four types, according to claim 10, wherein the counting of step (b) is performed by using the reagent as set forth in any one of claims 1-8.

12. A method of classifying leukocytes into five types, lymphocytes, monocytes, eosinophils, basophils and neutrophils, which comprises
(a) classifying the leukocytes into four types, lymphocytes, monocytes, eosinophils and granulocytes other than eosinophils, by the method as set forth in claim 10 or 11, and
(b) counting basophils by both the RF and DC methods or by the DC method alone using a reagent for the measurement of basophils which selectively leaves basophils intact.

13. A method of detecting abnormal cells by the RF and DC particle assay techniques using the reagent as set forth in any one of claims 1-8.

14. A method according to claims 9 and 13, by usinq the reagent as set forth in any one of claims 1-8.

## Patentansprüche

1. Reagens zum Klasifizieren von Leukocyten in einer Blutprobe, das Erythrocyten lysiert und auf Leukocyten wirkt, so daß die Klassifizierung und Zählung von Leukocyten ermöglicht wird, wobei sich das Reagens zusammensetzt aus:
(a) einem ersten Fluid, das ein Mittel zum Verdünnen von Blut ist und ein hyperosmotisches Mittel enthält;
(b) einem zweiten Fluid, das ein Tensid enthält, und zu einer Blutprobe zu geben ist, die mit dem ersten Fluid verdünnt ist, wobei das Tensid aus der Gruppe ausgewählt ist, bestehend aus
(i) einem Tensid einer ersten Gruppe, das ein anionisches Tensid auf Polyoxethylenbasis ist, dargestellt durch die Formel
R₁-R₂- (CH₂CH₂O)ₙ-X
worin R₁ eine Alkyl-, Alkenyl- oder Alkynylgruppe mit 10 bis 22 Kohlenstoffatomen; R₂ -O-, 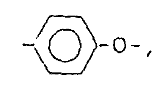 oder COO⁻; n eine ganze Zahl von 8 bis 30;
X -SO₃Na, COONa, OSO₃Na oder ONa sind; und
(ii) einem Tensid einer zweiten Gruppe, das ein nichtionisches Tensid auf Polyoxyethylenbasis ist, dargestellt durch die Formel:
R₁-R₂-(CH₂CH₂O)ₙ-H
worin R₁ eine Alkyl-, Alkenyl- oder Alkynylgruppe mit 10 bis 22 Kohlenstoffatomen; R₂ ist -O-, 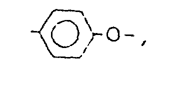 oder COO⁻; n eine ganze Zahl von 8 bis 30 sind;
worin das erste oder das zweite Fluid weiterhin ein Puffermittel enthält.

2. Reagens nach Anspruch 1, das ebenfals ein Tensid in dem ersten Fluid enthält.

3. Reagens nach Anspruch 1, worin das erste oder das zweite Fluid ein Löslichkeitsmittel enthält, das die Größe von Monocyten in Leukocyten selektiv vermindert.

4. Reagens zum Klassifizieren von Leukocyten in einer Blutprobe, das Erythrocyten lysiert und auf Leukocyten wirkt, wodurch die Klassifizierung und Zählung von Leukocyten ermöglicht wird, wobei das Reagens im wesentlichen aus einem Puffermittel, einem Löslichkeitsmittel, das selektiv die Größe von Monocyten in Leukocyten reduziert und zumindest einem Tensid wie in Anspruch 1 definiert besteht.

5. Reagens nach Anspruch 3 oder 4, worin das enthaltene Löslichkeitsmittel zumindest eine Verbindung ist, ausgewählt aus der Gruppe, bestehend aus den folgenden Verbindungen:
Harnstoff, Thioharnstoff, 1,1-Dimethylharnstoff, Ethylenharnstoff, Methylurethan, 1,3-Dimethylharnstoff, Urethan (H₂NCOOC₂H₅),n-Octyl-β-D-glucosid, CHAPS (3-[(3-Cholamidopropyl)dimethylammonio]-1-propansulfonat), CHAPSO (3-[(3-Cholamidopropyl)dimethylammonio]-2-hydroxy-1-propansulfonat), MEGA 8,9,10(Octanoyl-, Nonanoyl- oder Decanoyl-N-methylglucamid), Sucrosemonocaprat, N-Formylmethylleucylalanin, Guanidinthiocyanat, Guanylguanidin, Guanidinhydrochlorid, Guanidinrhodanat, Guanidinnitrat, 1,1,3,3-Tetraguanidin, Guanidincarbonat, Guanidinphosphat, Guanidinsulfat, Natriumdeoxychloat, Taurocholinsäure, Cholinsäure, Natriumtrichloracetat, Natriumtribromacetat, Natriumdichloracetat, Natriumdibromacetat, Natriummonochloracetat, Natriummonobromacetat.

6. Reagens nach Anspruch 4, das ein hyperosmotisches Mittel umfaßt.

7. Reagens zum Klassifizieren von Leukocyten, das sich aus den folgenden beiden Fluiden (a) und (b) zusammensetzt:
(a) einem ersten Fluid, wie in Anspruch 1 definiert; und
(b) einem zweiten Fluid, das das Reagens nach einem der Ansprüche 4 bis 6 enthält, und das zu einer Blutprobe zu geben ist, die mit dem ersten Fluid verdünnt ist.

8. Reagens nach Anspruch 7, das ebenfalls ein Tensid in dem ersten Fluid enthält.

9. Verfahren zum Klassifizieren von Leukocyten in Lymphocyten, Monocyten und Granulocyten durch (a) Ermittlung der Änderung der elektrischen Impedanz bei hoher Frequenz (RF-Verfahren) oder durch (b) Ermittlung der Änderung des Stromes aufgrund der Unterschiede der Leitfähigkeit zwischen suspendierten Teilchen und einem Fluidmedium, in dem sie suspendiert sind (DC-Verfahren), unter Verwendung des Reagens nach einem der Ansprüche 1 bis 8.

10. Verfahren zum Klassifizieren von Leukocyten in vier Typen, Lymphocyten, Monocyten, Eosinophilen und anderen Granulocyten als Eosinophilen, umfassend:
(a) Klassifizierung der Leukocyten in einer Blutprobe in drei Typen, Lymphocyten, Monocyten und Granulocyten, durch das Verfahren nach Anspruch 9, und
(b) Zählen der Anzahl von Eosinophilen, die nach der Passage einer vorbestimmten Zeitperiode in der Probe, die für Schritte (a) verwendet wird, selektiv intakt bleiben, durch das RF- und das DC-Verfahren oder durch das DC-Verfahren alleine.

11. Verfahren zum Klassifizieren von Leukocyten in vier Typen nach Anspruch 10, worin das Zählen von Schritt (b) unter Verwendung des Reagens nach einem der Ansprüche 1 bis 8 durchgeführt wird.

12. Verfahren zum Klassifizieren von Leukocyten in fünf Typen, Lymphocyten, Monocyten, Eosinophile, Basophile und Neutrophile, umfassend:
(a) Klassifizieren der Leukocyten in vier Typen, Lymphocyten, Monocyten, Eosinophile und andere Granulocyten als Eosinophile, durch das Verfahren nach Anspruch 10 oder 11, und
(b) Zählen von Basophilen durch das RF- und das DC-Verfahren oder durch das DC-Verfahren alleine unter Verwendung eines Reagens für die Messung von Basophilen, das Basophile selektiv intakt läßt.

13. Verfahren zum Ermitteln von abnormalen Zellen durch die RF- und DC-Teilchen-Assaytechniken unter Verwendung des Reagens nach einem der Ansprüche 1 bis 8.

14. Verfahren nach den Ansprüchen 9 und 13, unter Verwendung des Reagens nach einem der Ansprüche 1 bis 8.

## Revendications

1. Réactif pour la classification des leucocytes dans un échantillon de sang qui lyse les érythrocytes et qui agit sur les leucocytes pour permettre la classification et la numération des leucocytes, ledit réactif étant composé de :
(a) un premier fluide qui est un agent de dilution du sang et qui contient un agent hyperosmotique ;
(b) un deuxième fluide qui contient un tensioactif et qui est à ajouter à l'échantillon de sang qui a été dilué avec le premier fluide, ledit tensioactif étant sélectionné dans le groupe constitué par :
(i) un tensioactif d'un premier groupe qui est un tensioactif anionique à base de polyoxyéthylène représenté par la formule :
R₁-R₂-(CH₂CH₂O)ₙ-X
où R₁ un groupe alkyle, alcényle ou alcynyle ayant 10-22 atomes de carbone ; 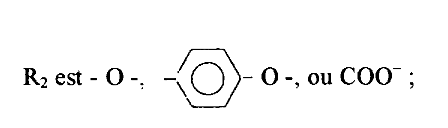 n est un entier de 8-30 ;
X est - SO₃Na, COONa, OSO₃Na ou ONa ; et
(ii) un tensioactif d'un deuxième groupe qui est un tensioactif non-ionique à base de polyoxyéthylène représenté par la formule :
R₁-R₂-(CH₂CH₂O)ₙ-H
où R₁ un groupe alkyle, alcényle ou alcynyle ayant 10-22 atomes de carbone ; 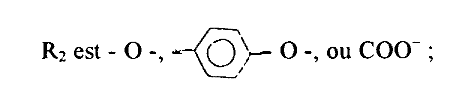 n est un entier de 8-30 ;
le premier ou le deuxième fluide contenant en outre un agent tampon.

2. Réactif tel que défini à la revendication 1, qui contient aussi un tensioactif dans le premier fluide.

3. Réactiftel que défini à la revendication 1, dans lequel ledit premier ou deuxième fluide contient un agent solubilisant qui réduit de façon sélective la taille des monocytes dans les leucocytes.

4. Réactif pour la classification des leucocytes dans un échantillon de sang qui lyse les érythrocytes et qui agit sur les leucocytes pour permettre la classification et la numération des leucocytes, ledit réactif consistant essentiellement en un agent tampon, un agent solubilisant qui réduit de façon sélective la taille des monocytes dans les leucocytes et au moins un tensioactiftel que défini à la revendication 1.

5. Réactif selon la revendication 3 ou 4, où l'agent solubilisant présent est choisi parmi au moins un membre du groupe constitué des suivants :
l'urée,
la thiourée,
la 1,1-diméthylurée
l'éthylèneurée
la méthylurethane
la 1,3-diméthylurée,
l'uréthane (H₂NCOOC₂H₅),
le N- octyl β - D - glucoside
le CHAPS : (3 - [(3 - cholamidopropyl) diméthylammonio] - 1 -propanesulfonate),
le CHAPSO : (3 - [(3 - cholamidopropyl) diméthylammonio] - 2 - hydroxy - 1 - propanesulfonate),
le MEGA : 8, 9, 10 (octanoyl -, nonanoyl - ou décanoyl -N - méthylglucamide),
le monocaprate de saccharose,
la N - formylméthylleucylalanine,
le thiocyanate de guanidine,
La guanylguanidine,
le chlorhydrate de guanidine,
le rhodanate de guanidine,
le nitrate de guanidine,
la 1, 1, 3, 3 - tetraguanidine,
le carbonate de guanidine,
le phosphate de guanidine,
le sulfate de guanidine,
le déoxycholate de sodium,
l'acide taurocholique,
l'acide cholique,
le trichloroacétate de sodium,
le tribromoacétate de sodium,
le dichloroacétate de sodium,
le dibromoacétate de sodium,
le monochloroacétate de sodium,
le monobromoacétate de sodium.

6. Réactif selon la revendication 4, qui contient un agent hyperosmotique.

7. Réactif pour la classification des leucocytes qui est composé des deux fluides suivants (a) et (b) :
(a) un premier fluide tel quedéfini à la revendication 1 ; et
(b) un deuxième fluide qui contient un réactif selon l'une quelconque des revendications 4 à 6 et qui est à ajouter à l'échantillon de sang qui a été dilué à l'aide du premier fluide.

8. Réactif selon la revendication 7, qui contient aussi un tensioactif dans le premier fluide.

9. Procédé de classement des leucocytes en lymphocytes, monocytes et granulocytes par (a) détection de la variation d'impédance électrique à haute fréquence (procédé RF) ou par (b) détection de la variation de courant due aux différences de conductivité entre les particules en suspension et un milieu fluide dans lesquelles elles sont mises en suspension (procédé DC) en utilisant un réactif selon l'une quelconque des revendications 1 à 8.

10. Procédé de classement des leucocytes en quatre types, lymphocytes, monocytes, éosinophiles et granulocytes autres que les éosinophiles, qui comprend (a) le classement des leucocytes d'un échantillon de sang en trois types, lymphocytes, monocytes et granulocytes, par le procédé selon la revendication 9, et (b) le comptage par les deux procédés RF et DC ou par le seul procédé DC du nombre d'éosinophiles qui restent intacts de manière sélective dans l'échantillon qui a été utilisé pour l'étape (a) après l'écoulement d'une période de temps prédeterminée.

11. Procédé de classement des leucocytes en quatre types, selon la revendication 10, où le comptage de l'étape (b) est effectué en utilisant un réactif selon l'une quelconque des revendications 1 à 8.

12. Procédé de classification de leucocytes en cinq types, lymphocytes, monocytes, éosinophiles, basophiles et neutrophiles, qui comprend
(a) le classement des leucocytes en quatre types, lymphocytes, monocytes, éosinophiles et granulocytes autre que des éosinophiles, par un procédé selon la revendication 10 ou 11, et
(b) la numération des basophiles à l'aide des deux procédés RF et DC ou à l'aide du seul procédé DC en utilisant un réactif pour la mesure des basophiles qui laisse les basophiles intacts de manière sélective.

13. Procédé de détection de cellules anormales à l'aide des procédés RF et DC d'analyse de particules en utilisant un réactif selon l'une quelconque des revendications 1 à 8.

14. Procédé selon les revendications 9 et 13, mettant en oeuvre un réactif selon l'une quelconque des revendications 1 à 8.
